(19) Europäisches Patentamt
European Patent Office
Office européen des brevets

(11) EP 0 770 059 B1

(12) **EUROPÄISCHE PATENTSCHRIFT**

(45) Veröffentlichungstag und Bekanntmachung des Hinweises auf die Patenterteilung: **07.04.1999 Patentblatt 1999/14**

(21) Anmeldenummer: **95921824.9**

(22) Anmeldetag: **03.06.1995**

(51) Int Cl.[6]: **C07C 259/18**, C07C 271/64, C07C 257/20, C07C 257/18, A61K 31/155

(86) Internationale Anmeldenummer: **PCT/EP95/02112**

(87) Internationale Veröffentlichungsnummer: **WO 96/02497 (01.02.1996 Gazette 1996/06)**

(54) **SUBSTITUIERTE BENZAMIDINE, IHRE HERSTELLUNG UND VERWENDUNG ALS ARZNEIMITTEL**

SUBSTITUTED BENZAMIDINES, THEIR PRODUCTION AND THEIR USE AS MEDICAMENTS

BENZAMIDINES SUBSTITUEES, LEUR PREPARATION ET LEUR UTILISATION COMME MEDICAMENTS

(84) Benannte Vertragsstaaten:
**AT BE CH DE DK ES FR GB GR IE IT LI LU MC NL PT SE**
Benannte Erstreckungsstaaten:
**LT LV SI**

(30) Priorität: **13.07.1994 DE 4424713**

(43) Veröffentlichungstag der Anmeldung: **02.05.1997 Patentblatt 1997/18**

(73) Patentinhaber: **Boehringer Ingelheim Pharma KG**
**55216 Ingelheim am Rhein (DE)**

(72) Erfinder:
- **ANDERSKEWITZ, Ralf**
  **D-55411 Bingen (DE)**
- **SCHROMM, Kurt**
  **D-55218 Ingelheim (DE)**
- **RENTH, Ernst-Otto**
  **D-55218 Ingelheim (DE)**
- **BIRKE, Franz**
  **D-55218 Ingelheim (DE)**
- **FÜGNER, Armin**
  **D-55435 Gau-Algesheim (DE)**
- **HEUER, Hubert**
  **D-55270 Schwabenheim (DE)**
- **MEADE, Christopher**
  **D-55411 Bingen (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 518 818**
**EP-A- 0 518 819**
**EP-A- 0 601 977**
**WO-A-93/16036**
**WO-A-94/11341**

Bemerkungen:
Die Akte enthält technische Angaben, die nach dem Eingang der Anmeldung eingereicht wurden und die nicht in dieser Patentschrift enthalten sind.

EP 0 770 059 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft neue substituierte Benzamidine, ihre Herstellung nach konventionellen Methoden und ihre Verwendung als Arzneistoffe mit insbesondere $LTB_4$-rezeptorantagonistischer Wirkung.

Die neuen substituierten Benzamidine entsprechen der Formel

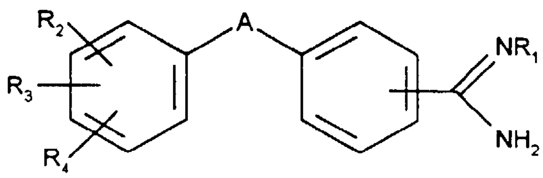

(I)

in der

A für die Gruppe

$$-X_1-C_mH_{2m}-X_2- \qquad (II)$$

(m gleich 2 bis 6)
oder

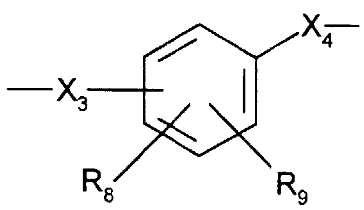

(III)

steht;

$X_1$ für O, NH oder $NCH_3$

$X_2$ für O, NH, $NCH_3$ oder

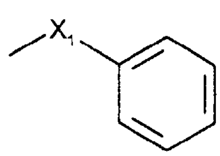

(IV)

$X_3$ für $-X-C_nH_{2n}-$;

$X_4$ für $-C_nH_{2n}-X-$ (n = 1 oder 2, X = O, NH oder $NCH_3$);

$R_1$ für OH, CN, $COR_{12}$, $COOR_{12}$ und CHO;

$R_2$ für Br, Cl, F, $CF_3$, OH, $C_1$-$C_6$-Alkyl, $C_5$-$C_7$-Cycloalkyl, Aryl, O-Aryl, $CH_2$-Aryl, $CR_5R_6$-Aryl, $C(CH_3)_2$-$R_7$;

außerdem für H, wenn A ein Rest der Formel III oder wenn $X_2$ ein Rest der Formel IV ist;
außerdem für $C_1$-$C_6$-Alkoxy, wenn A die Gruppe II ist, $X_1$ und m die obige Bedeutung haben und $X_2$ für NH, $NCH_3$ oder die Gruppe IV steht,
oder wenn A die Gruppe III bedeutet, $X_3$ die obige Bedeutung hat und in $X_4$ X für NH oder $NCH_3$ steht;

$R_3$ für H, $C_1$-$C_6$-Alkyl, OH, Cl, F, außerdem für $C_1$-$C_6$-Alkoxy, wenn $R_2$ Aryl, O-Aryl, $CH_2$-Aryl, $CR_5R_6$-Aryl oder $C(CH_3)_2$-$R_7$ ist oder wenn $X_2$ die Gruppe IV bedeutet;
$R_2$ und $R_3$ können gemeinsam auch einen ankondensierten aromatischen oder oder heteroaromatischen Ring bedeuten;

$R_4$ für H oder $C_1$-$C_6$-Alkyl;

$R_5$ für $C_1$-$C_4$-Alkyl, $CF_3$, $CH_2OH$, COOH oder COO($C_1$-$C_4$-Alkyl);

$R_6$ für H, $C_1$-$C_4$-Alkyl oder $CF_3$;
$R_5$ und $R_6$ können gemeinsam auch eine $C_4$-$C_6$-Alkylengruppe bilden;

$R_7$ für $CH_2OH$, COOH, COO($C_1$-$C_4$-Alkyl), $CONR_{10}R_{11}$ oder $CH_2NR_{10}R_{11}$;

$R_8$, $R_9$ für H, Br, Cl, F, OH, $C_1$-$C_6$-Alkyl oder $C_1$-$C_6$-Alkoxy;

$R_{10}$ für H, $C_1$-$C_6$-Alkyl, Phenyl, Phenyl-($C_1$-$C_6$-Alkyl), $COR_{12}$, $COOR_{12}$, CHO, $CONH_2$, $CONHR_{12}$, $SO_2$-($C_1$-$C_6$-Alkyl), $SO_2$-Phenyl,
wobei der Phenylrest ein- oder mehrfach durch Cl, F, $CF_3$, $C_1$-$C_4$-Alkyl, OH, $C_1$-$C_4$-Alkoxy substituiert sein kann;

$R_{11}$ H oder $C_1$-$C_6$-Alkyl;
$R_{10}$ und $R_{11}$ können gemeinsam auch eine $C_4$-$C_6$-Alkylengruppe darstellen;

$R_{12}$ für $C_1$-$C_6$-Alkyl, $C_5$-$C_7$-Cycloalkyl, Aryl, Heteroaryl, Aralkyl oder Heteroaryl-($C_1$-$C_6$-Alkyl), wobei die Aryl- oder Heteroarylgruppen ein- oder mehrfach durch Cl, F, $CF_3$, $C_1$-$C_4$-Alkyl, OH oder $C_1$-$C_4$-Alkoxy substituiert sein können

mit der Maßgabe, daß im Falle

A für eine Gruppe -$X_1$-$C_mH_{2m}$-$X_2$- steht,
worin m eine ganze Zahl 2,3 oder 4 und

$X_1$ O, NH,

$X_2$ O, NH oder

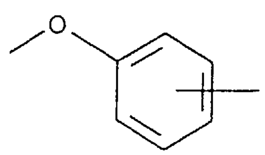

und

$R_2$ Wasserstoff, Br, Cl, F, $CF_3$, $C_1$-$C_6$-Alkyl, Phenyl,

$R_3$ Wasserstoff, $C_1$-$C_6$-Alkyl, Hydroxy, Cl, F, $C_1$-$C_6$-Alkoxy,

$R_4$ Wasserstoff, $C_1$-$C_6$-Alkyl

bedeuten,

$R_1$ nicht die Bedeutung von -OH haben darf.

Die neuen Verbindungen können jeweils als freie Basen oder als Salze mit Säuren, vorzugsweise mit physiologisch verträglichen Säuren, vorliegen; soweit sie ein oder mehrere chirale Zentren enthalten, werden sie als Racemate, in enantiomerenreiner bzw. angereicherter Form, ggf. als Diastereomerenpaare, beansprucht. Eingeschlossen sind auch etwaige Tautomere (mit -C(NH)-$NHR_1$).

Bevorzugt sind Verbindungen der Formel I, worin

| | |
|---|---|
| A, m, n, $X_3$, $X_4$ und $R_1$ | die obige Bedeutung haben; |
| $X_1$ | für O; |
| $X_2$ | für O oder eine Gruppe IV (worin $X_1$ aleich O ist); |
| $R_2$ | für Cl, F, $CF_3$, OH, $C_1$-$C_6$-Alkyl, Aryl, O-Aryl, $CH_2$-Aryl oder $CR_5R_6$-Aryl, und, wenn $X_2$ die Gruppe IV ist, außerdem für $C_1$-$C_6$-Alkoxy; |
| $R_3$ | für H, $C_1$-$C_6$-Alkyl oder OH, und, wenn $R_2$ $CR_5R_6$-Aryl ist, außerdem für $C_1$-$C_6$-Alkoxy; |
| $R_4$ | für H; |
| $R_5$ | für $C_1$-$C_3$-Alkyl, $CF_3$ oder $CH_2OH$; |
| $R_6$ | für H, $C_1$-$C_3$-Alkyl oder $CF_3$, $R_5$ und $R_6$ gemeinsam auch für $C_4$-$C_5$-Alkylen; |
| $R_8$, $R_9$ | für H, F oder OH |

stehen.

Besonders hervorzuheben sind die Verbindungen, bei denen im Rahmen der vorstehenden Definitionen

| | |
|---|---|
| $X_1$ | für O; |
| $X_2$ | für die Gruppe IV (worin $X_1$ gleich O ist); |
| X | für O; |
| $R_1$ | für $COOR_{12}$; |
| $R_2$ | für $C_1$-$C_6$-Alkyl, Aryl, O-Aryl, $CH_2$-Aryl oder $CR_5R_6$-Aryl; |
| $R_3$ | für H, OH oder $C_1$-$C_6$-Alkyl und, wenn $R_2$ $CR_5R_6$-Aryl ist, auch für $C_1$-$C_6$-Alkoxy; |
| $R_4$ | für H; |
| $R_5$, $R_6$ | für $C_1$-$C_3$-Alkyl oder $CF_3$; |
| $R_8$,$R_9$ | für H; |
| $R_{12}$ | für $C_1$-$C_6$-Alkyl, Aralkyl oder $C_7$-$C_5$-Cycloalkyl steht. |

Soweit die Symbole nach den obigen Definitionen gleiche oder verschiedene Bedeutungen haben können, sollen beide Möglichkeiten eingeschlossen sein. Aliphatische Ketten, die eine ausreichende Zahl von C-Atomen aufweisen, können geradkettig oder verzweigt sein.

"Aryl" bedeutet einen gegebenenfalls (ein- oder mehrfach) substituierten Arylrest, etwa Naphthyl, vorzugsweise jedoch einen gegebenenfalls (ein- oder mehrfach) substituierten Phenylrest. Substituenten sind bevorzugt: Cl, F, Br, OH, $C_1$-$C_6$-Alkyl, $C_1$-$C_6$-Alkoxy, $CF_3$; möglich sind außerdem Gruppen, die in der Regel nur einfach vorhanden sind, wie $NH_2$, NH($C_1$-$C_6$-Alkyl), N($C_1$-$C_6$-Alkyl)$_2$, NH($SO_2$-($C_1$-$C_6$-Alkyl)), NH($SO_2$-Phenyl), wobei der Phenylrest seinerseits insbesondere durch F, Cl, $CF_3$, $C_1$-$C_4$-Alkyl, $C_1$-$C_4$-Alkoxy oder OH substituiert sein kann.

Mit "Aralkyl" wird eine $C_1$-$C_6$-Alkylgruppe bezeichnet, die durch ein Aryl (wie oben definiert) substituiert ist.

Unter "Heteroaryl" werden hier bevorzugt Pyridyl, Pyrimidyl, Pyridazinyl und Pyrazinyl, Thienyl und Furyl verstanden, die insbesondere durch Cl, F, Br, OH, $C_1$-$C_6$-Alkyl, $C_1$-$C_6$-Alkoxy, $CF_3$ ein- oder mehrfach substituiert sein können. Die Cycloalkylgruppen können durch $C_1$-$C_6$-Alkylgruppen substituiert sein, z. B. wie im Menthyl.

Sofern $R_2$ und $R_3$ gemeinsam einen ankondensierten Ring bedeuten, ist damit ein Ring gemeint, wie er oben für Aryl und Heteroaryl zugrunde gelegt wurde.

Bei den Alkyl- und Alkoxygruppen liegt die Zahl der C-Atome bevorzugt zwischen 1 und 4. Als Substituenten in aromatischen bzw. heteroaromatischen Resten sind Alkyl und Alkoxygruppen mit bis zu 3, vor allem mit bis zu 2 C-Atomen, bevorzugt. Die Amidinogruppe C($NH_2$)$NR_1$ befindet sich bevorzugt in para-Position zu der Gruppe A.

Die neuen Verbindungen können nach an sich bekannten Verfahren, beispielsweise wie folgt, erhalten werden:

1. Umsetzung eines Amidins der Formel

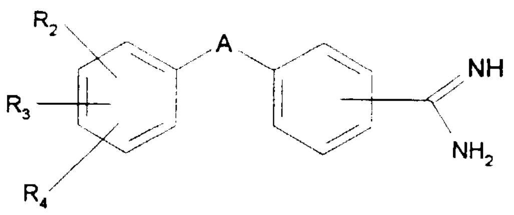

(V)

mit einer Verbindung der Formel

$$L - R'_1 \qquad \text{(VI)}$$

wobei in (V) A, $R_2$, $R_3$ und $R_4$ die obige Bedeutung haben, $R'_1$ dieselbe Bedeutung wie $R_1$ hat, ausgenommen OH, und L eine nucleophile Austrittsgruppe wie ein Halogenatom (etwa Cl, Br) oder Acyloxy darstellt.

Die Umsetzung wird zweckmäßigerweise in einem Lösungsmittel wie Tetrahydrofuran, Methylenchlorid, Chloroform oder Dimethylformamid, vorzugsweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt.

2. Umsetzung von Verbindungen der Formel

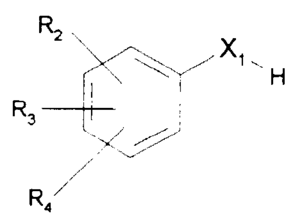

(VII)

(worin $R_2$, $R_3$, $R_4$ und $X_1$ die obige Bedeutung haben), mit einem Benzamidinderivat der Formel

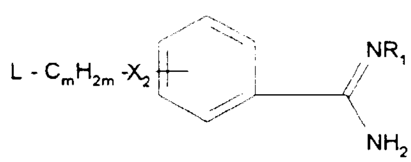

(VIII)

oder

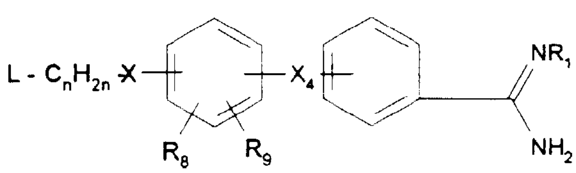

(IX)

(worin L, m, n, X, $X_2$, $X_4$, $R_1$, $R_8$ und $R_9$ die obige Bedeutung haben).

3. Umsetzung von Verbindungen der Formel

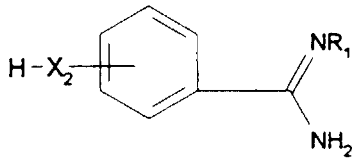

(X)

($R_1$ und $X_2$ in der obigen Bedeutung) mit einer Verbindung der Formel

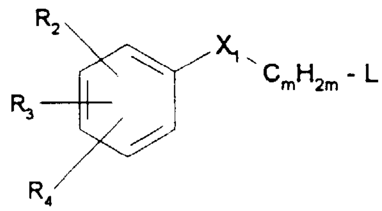

(XI)

oder

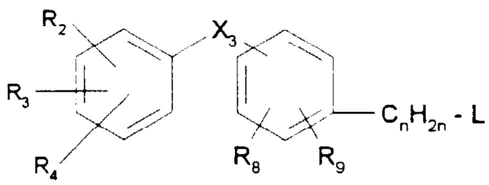

(XII)

(worin L, m, n, $R_2$, $R_3$, $R_4$, $R_8$, $R_9$, $X_1$ und $X_3$ die obige Bedeutung haben).

Verfahren 2 und 3 werden zweckmäßig in aprotischen Lösungsmitteln wie Dimethylsulfoxid, Dimethylformamid, Acetonitril oder Alkoholen (z.B. Methanol, Ethanol, Isopropanol) unter Zusatz von basischen Stoffen (etwa Metallcarbonate, Metallhydroxide, Metallhydride) bei Temperaturen zwischen etwa 0 und 140°C, bzw. bei der Siedetemperatur des Reaktionsgemischs durchgeführt.

4. Zur Herststellung von Verbindungen der Formel I, in denen $R_1$ OH bedeutet: Umsetzung von Nitrilen der Formel

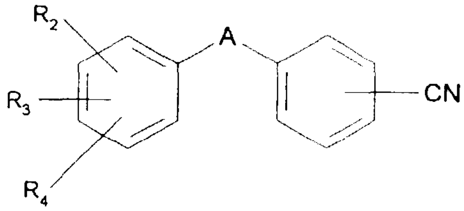

(XIII),

worin A, $R_2$, $R_3$, und $R_4$ die obige Bedeutung haben, mit Hydroxylamin.

Verfahren 4 wird zweckmäßig in einem Alkohol (Methanol, Ethanol, Propanol) oder einem aprotischen Lösungsmittel wie Dimethylsulfoxid, Dimethylformamid oder Acetonitril, ggf in Mischung mit Wasser, in der Wärme durchgeführt. Das Hydroxylamin wird z.B. als Hydrochlorid oder als Methansulfonat eingesetzt und eine geeignete Base, etwa Natriumcarbonat zugegeben

Die Ausgangsstoffe können nach üblichen Methoden synthetisiert werden.

Aus dem Stand der Technik - so zum Beispiel aus den Europäischen Patentanmeldungen EP-A-0 515 818, EP-A-0 518 819 und der EP-A-0 601 977 sowie aus der Internationalen Patentanmeldung WO-A-93/16036 - sind zwar

strukturähnliche Verbindungen mit gleicher Wirkungsrichtung bekannt, jedoch sind aus dem Stand der Technik keine Verbindungen bekannt, die eine verbesserte $LTB_4$-antagonistische Wirkung bei oraler Applikation aufweisen.

Wie gefunden wurde, zeichnen sich die Verbindungen der Formel I durch vielfältige Anwendungsmöglichkeiten auf therapeutischem Gebiet aus. Hervorzuheben sind solche Anwendungsmöglichkeiten, für welche die $LTB_4$-rezeptorantagonistischen Eigenschaften eine Rolle spielen. Hier sind insbesondere zu nennen:
Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, Alzheimer-Krankheit, Schock, Reperfusionsschäden/Ischämien, Atherosklerose, Multiple Sklerose.
Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des $LTB_4$ oder eines anderen Moleküls (beispielsweise 12-HETE) mit dem $LTB_4$-Rezeptor einen Einfluß auf die Zell-Proliferation hat (etwa chronische myelozytische Leukämie).
Die neuen Verbindungen können auch in Kombination mit anderen Wirkstoffen angewendet werden, etwa solchen, die für dieselben Indikationen Verwendung finden, oder z.B. mit Antiallergika, Sekretolytika, $\beta_2$-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Die neuen Verbindungen zeichnen sich durch gute Verträglichkeit und günstige Bioverfügbarkeit aus.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt. Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragees, Kapseln, Oblaten, Pulver, Granulate, Lösungen, Emulsionen, Sirupe, Inhalationsaerosole, Salben, Suppositorien.

Zur pharmakologischen und biochemischen Untersuchung der Wirkungsverhältnisse eigenen sich Tests, wie sie beispielsweise in der WO 93/16036, S. 15 bis 17, dargestellt sind.

<u>Formulierungsbeispiele</u>

| 1. Tabletten | |
|---|---|
| Zusammensetzung: | |
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

| 2. Suppositorien | |
|---|---|
| Zusammensetzung: | |
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

3. <u>Inhalationspulver</u>

Mikronisiertes Wirkstoffpulver (Verbindung der Formel I; Teilchengröße ca. 0,5 bis 7 μm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, inhaliert.

Die nachstehenden Beispiele geben Anhaltspunkte für die Herstellung der neuen Verbindungen.

Beispiel 1

(Methoxycarbonyl-imino-{4'-[2-(2-propylphenoxy)-ethoxy]-biphenyl-4-yl}-methyl)-amin

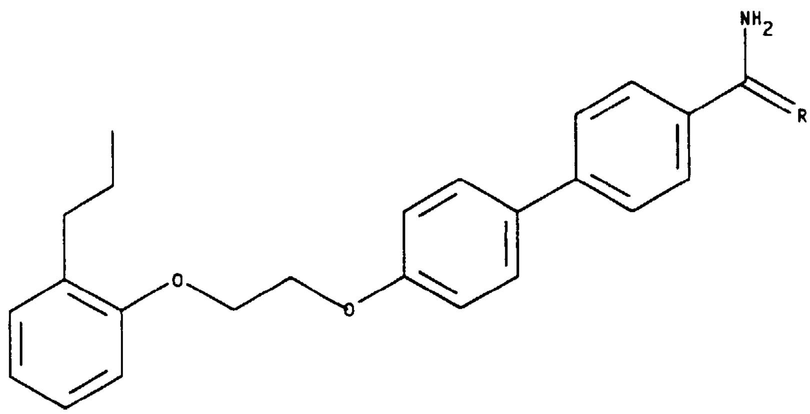

3,8 g der Amidinverbindung der obigen Formel (R gleich NH), die nach üblichen Methoden erhalten werden kann, z. B. nach den Verfahren gemäß WO 93/16036, werden in 200 ml Chloroform suspendiert. 1,6 ml Triethylamin werden zugegeben und 0,8 ml Chlorameisensäuremethylester bei Raumtemperatur zugetropft. Nach Lösung der Komponenten wird 3 Stunden gerührt, danach dreimal mit Wasser ausgeschüttelt, eingedampft und der Rückstand mit Ether verrührt und abgesaugt. Ausbeute an Verbindung der obigen Formel mit R gleich $NCOOCH_3$ : 3,7 g, Fp. 170-6°C.

Beispiel 2

(Benzyloxycarbonyl-imino-{4"-[2-(2-propylphenoxy)-ethoxy]-biphenyl-4-yl}methyl)amin

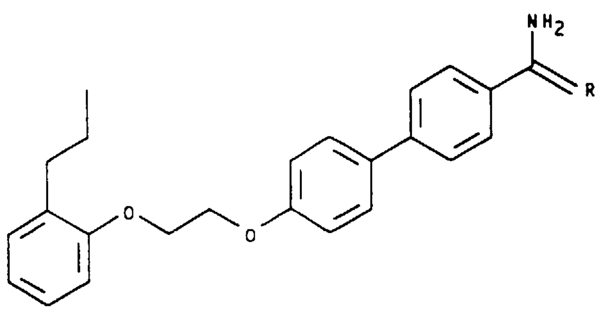

2,6 g der Amidinverbindung der obigen Formel (R = NH) werden in 200 ml Chloroform vorgelegt. 1,3 ml Triethylamin werden zugegeben und 1 ml Chlorameisensäurebenzylester bei Raumtemperatur zugetropft. Nach Lösung der Komponenten wird
3 Stunden gerührt, danach dreimal mit Wasser ausgeschüttelt, eingedampft und der Rückstand mit Ether verrührt und abgesaugt. Die Substanz wird aus Ethanol umkristallisiert. Ausbeute der Verbindung der obigen Formel mit R = $NCOOCH_2Ph$: 2,2 g, Fp. 128-131°C.

Entsprechend hergestellte Verbindungen mit anderen Resten R:

R = $NCOOC_2H_5$; Fp. 120 - 123 °C
R = NCOO-n-$C_3H_7$; Fp. 113 - 114°C
R = NCOO-i-$C_3H_7$; Fp. 110 - 117°C
R = NCOO-n-$C_4H_9$; Fp. 135 - 138°C
R = NCOO-i-$C_4H_9$; Fp. 103°C
R = NCOO-t-$C_4H_9$; Fp. 129 - 132°C
R = NCOO-n-$C_6H_{13}$; Fp. 117 - 121°C

Beispiel 3

3,5 g der Amidinverbindung der obigen Formel (R = NH) werden in 150 ml Chloroform vorgelegt. 2 ml Triethylamin werden zugegeben und 1 ml Di-tert.-butyldicarbonat bei Raumtemperatur zugetropft. Nach Lösung der Komponenten wird 3 Stunden gerührt, danach dreimal mit Wasser ausgeschüttelt, eingedampft und der Rückstand mit Ether verrührt und abgesaugt. Die Substanz wird aus 20 ml Ethanol umkristallisiert. Ausbeute der Verbindung der obigen Formel mit R = N-COO-t-Butyl: 3 g, Fp. 129-132°C.

Beispiel 4

(a) [Hydroxy-imino-(4-{3-[4-(1-methyl-1-phenylethyl)-phenoxymethyl]-benzyloxy}phenyl)-methyl]-amin

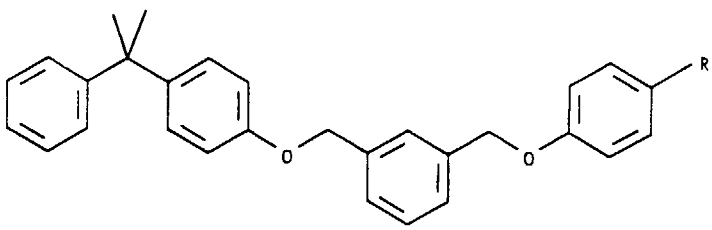

5,25 g Nitril der obigen Formel (R = CN, hergestellt nach üblichen Methoden) werden in 60 ml Ethanol vorgelegt und zum Sieden erhitzt. Im Lauf von 30 Min. wird eine Lösung von 2,7 g Natriumcarbonat und 3,4 g Hydroxylaminhydrochlorid in 10 ml Wasser zugetropft. Anschließend wird 5 Stdn. unter Rückfluß gekocht. Die Mischung wird nach dem Erkalten eingeengt, der Rückstand in 50 ml Wasser aufgenommen und dreimal mit je 40 ml Essigester extrahiert. Die organischen Phasen werden über $MgSO_4$ getrocknet, filtriert und eingeengt. Die Kristalle werden in 20 ml Aceton aufgenommen und mit etherischer Salzsäure angesäuert. Nach kurzem Lösen bilden sich 5,3 g des Hydrochlorids des Amidoxims der obigen Formel mit R = C(NOH)-$NH_2$. Fp. 180-181°C.

(b) [Imino-(4-{3-[4-(1-methyl-1-phenylethyl)-phenoxymethyl]-benzyloxy}phenyl)-methyl]-amin

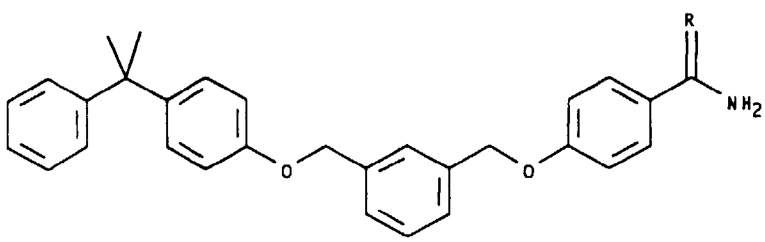

5,1 g des Amidoxims der obigen Formel (R = NOH) werden in 120 ml Methanol gelöst und mit 10 g methanolfeuchtem Raney-Nickel 2 Stdn. bei Normaldruck und Raumtemperatur hydriert. Das Nickel wird abgesaugt und die Lösung über Kieselgur filtriert. Das Filtrat wird mit ethanolischer Salzsäure angesäuert, die Lösung eingeengt und aus Ethanol umkristallisiert. Die Ausbeute beträgt 3,3 g der Amidinverbindung (obige Formel, R = NH). Fp. 160°C.

(c) [Ethoxycarbonyl-imino-(4-{3-[4-(1-methyl-1-phenylethyl)-phenoxymethyl]-benzyloxy}phenyl)-methyl]-amin

2,44 g der nach (b) erhaltenen Amidinverbindung der obigen Formel (R gleich NH) werden in 150 ml Dichlormethan vorgelegt, 0,6 g Chlorameisensäureethylester werden zugegeben, dann werden bei Raumtemperatur 52,5 ml 0,2 N Natronlauge in 15 Minuten zugetropft. Die entstehende Lösung wird 2 Stdn. bei Raumtemperatur gerührt. danach wird die organische Phase abgetrennt, mit 100 ml Wasser extrahiert und über Natriumsulfat getrocknet. Die Lösung wird eingeengt und der Rückstand aus 10 ml Ethanol umkristallisiert. Man erhält 2,1 g der Titelverbindung (R = $NCOOC_2H_5$), Fp. 99°C.

Analog können beispielweise folgende Verbindungen der in Beispiel 4(b) angegebenen Formel erhalten werden:

R = NCOO-(-)-Menthyl; Fp. 113°C
R = NCO-$C_6H_5$; Fp. 101 - 103°C

Beispiel 5

[3'-Pyridylcarbonyl-imino-(4-{3-[4-(1-methyl-1-phenylethyl)-phenoxymethyl]-benzyloxy}phenyl)-methyl]-amin

5.0 g der Amidinverbindung der obigen Formel (R = NH, vgl. Bsp. 4(b)) werden in 250 ml Dichlormethan vorgelegt. Innerhalb von 10 Min. wird bei Raumtemperatur eine Lösung von 3,9 g Nicotinsäurechlorid-hydrochlorid und 16,3 ml Triethylamin in 50 ml Dichlormethan zugetropft. Nach 15 Stdn. bei Raumtemperatur wird zweimal mit je 300 ml Wasser extrahiert, die organische Phase über Natriumsulfat getrocknet, filtriert und das Filtrat eingeengt. Der Rückstand wird über Kieselgel 60 in Essigester niederdruckchromatographisch gereinigt, das Produkt in 50 ml Aceton gelöst, mit ethanolischer Salzsäure angesäuert und mit Ether als Hydrochlorid ausgefällt. Die Ausbeute beträgt 2,0 g des Nicotinoylderivats der obigen Formel mit R = N-CO-3-Pyridyl vom Fp. 172°C.

Entsprechend den Beispielen können u. a. auch die folgenden Verbindungen hergestellt werden:

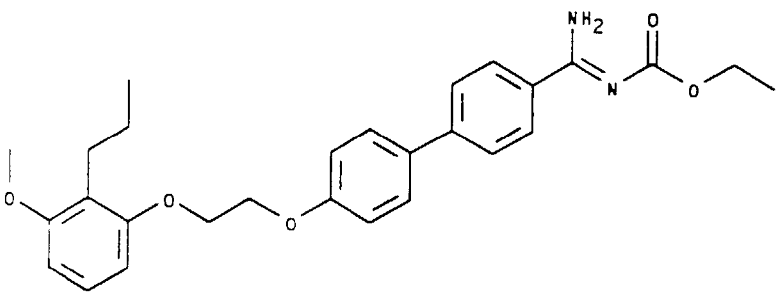

107-111°C

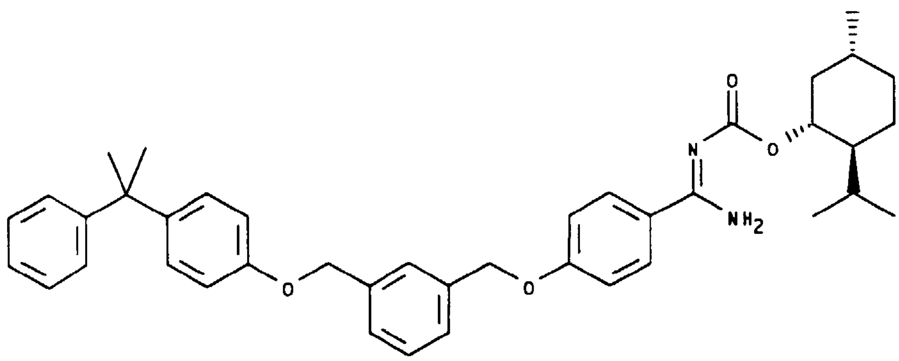

(-)-Menthylester

113°C

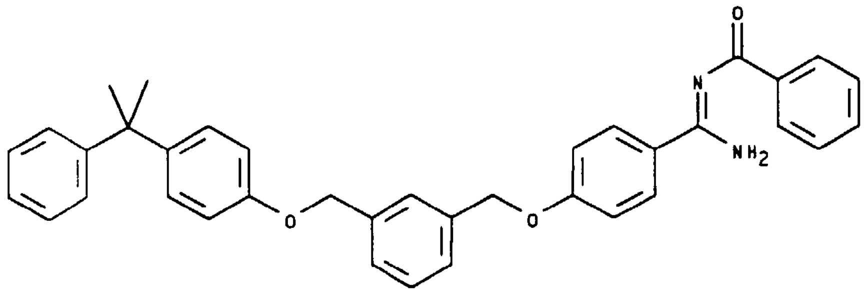

101-103°C

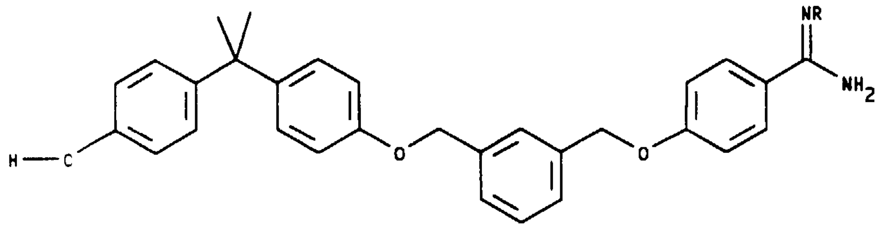

R = OH Fp. 181°C
R = $COOC_2H_5$ Fp. 131°C (mit 1mol Ethanol)

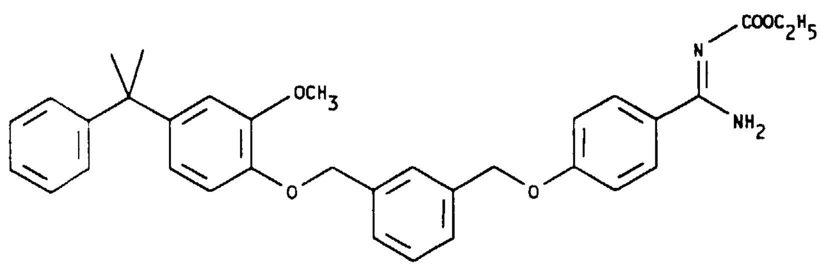

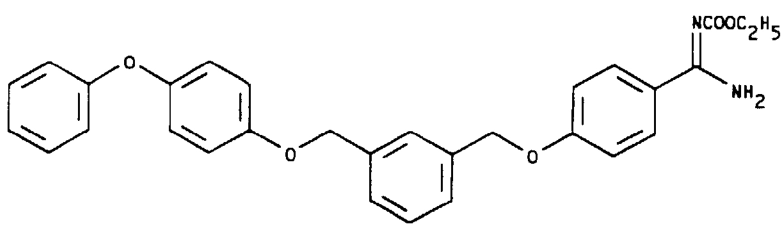

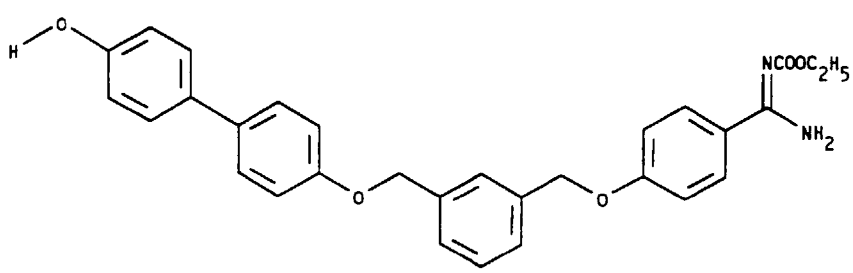

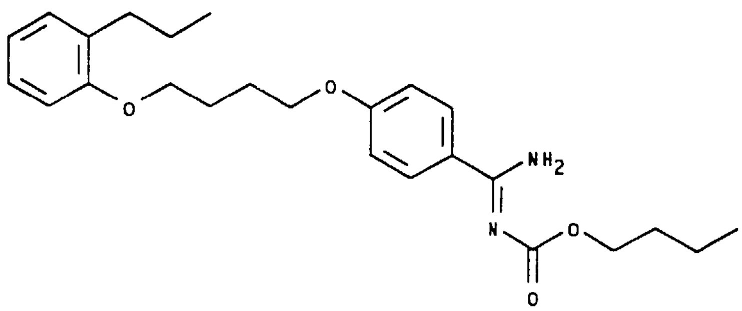

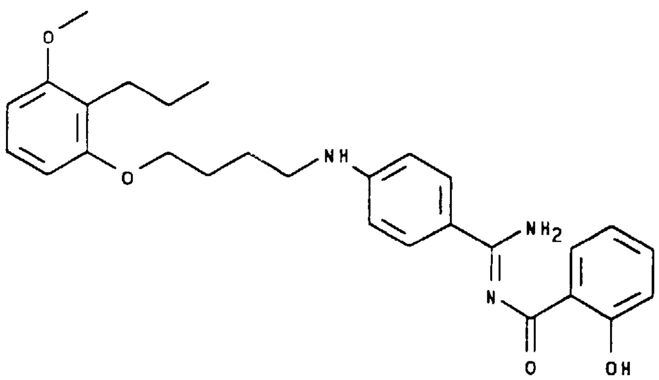

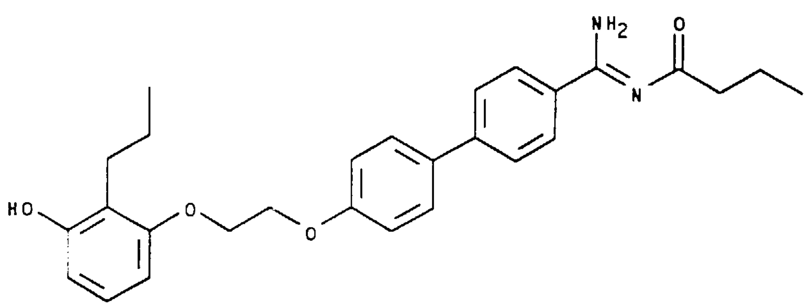

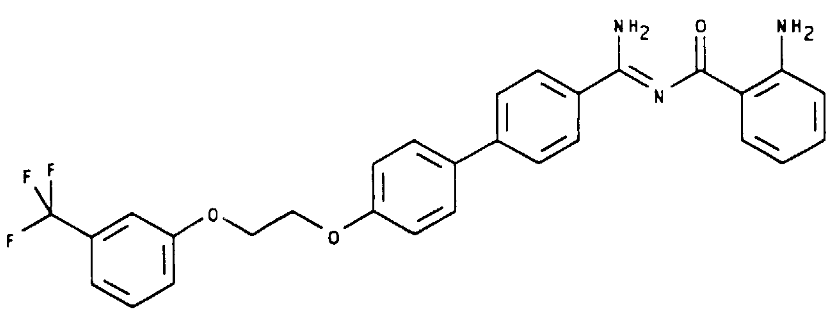

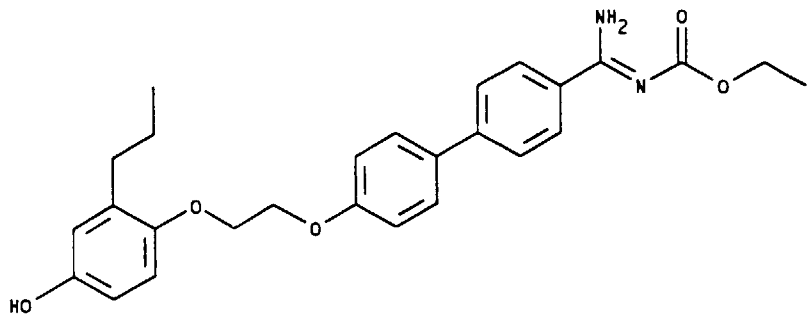

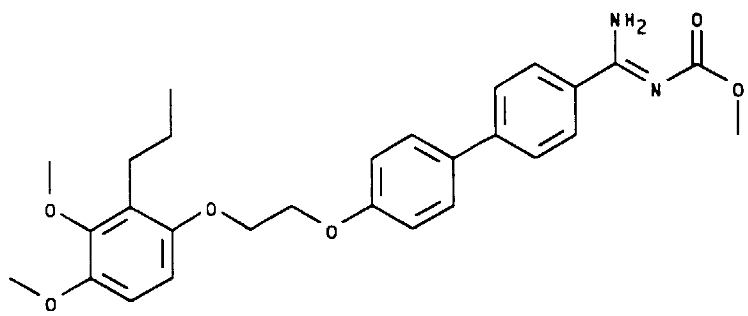

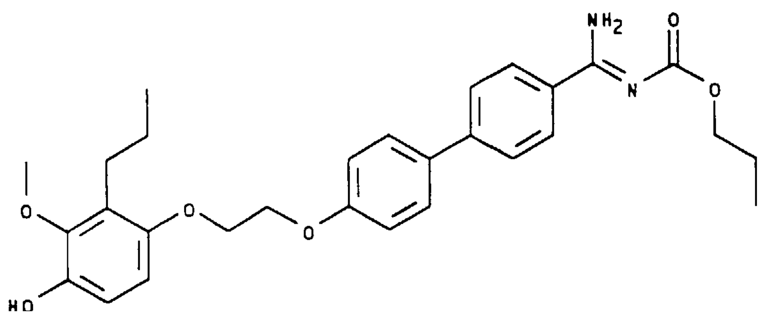

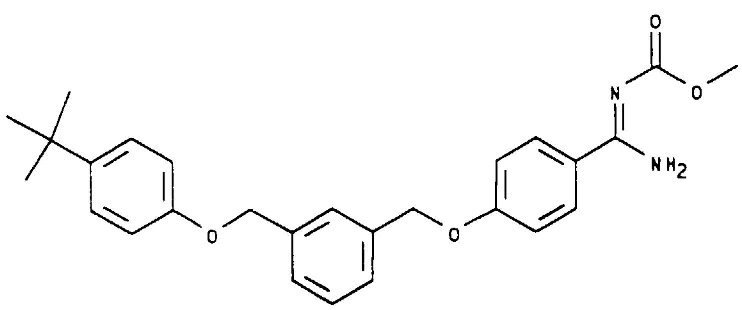

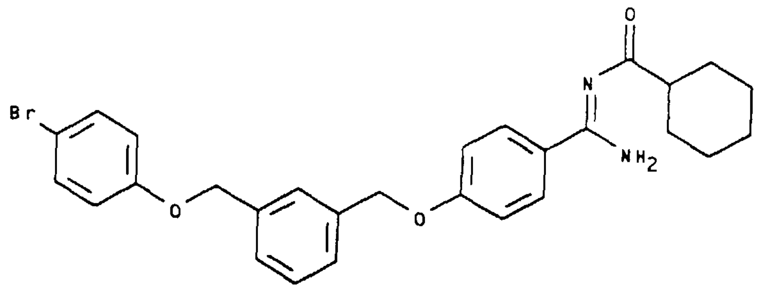

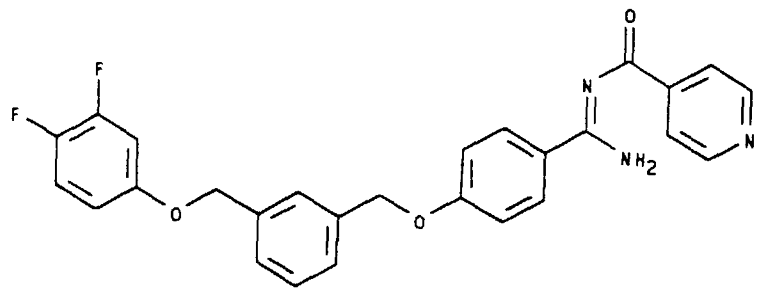

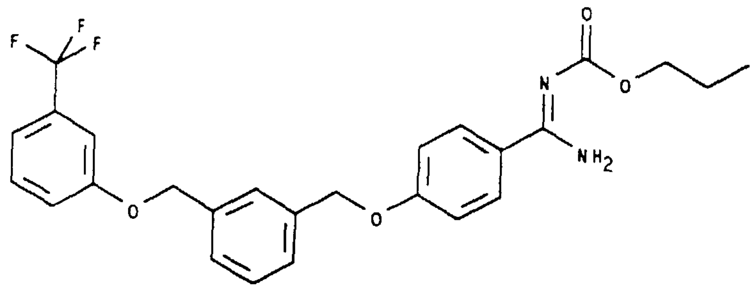

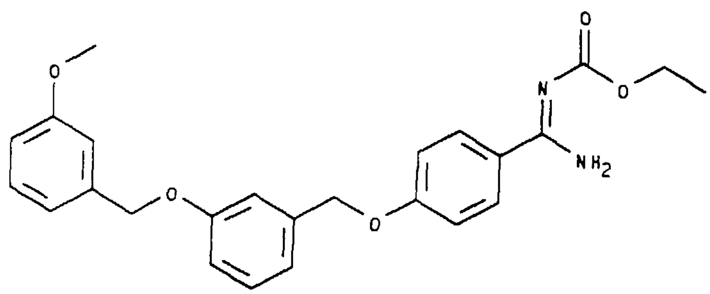

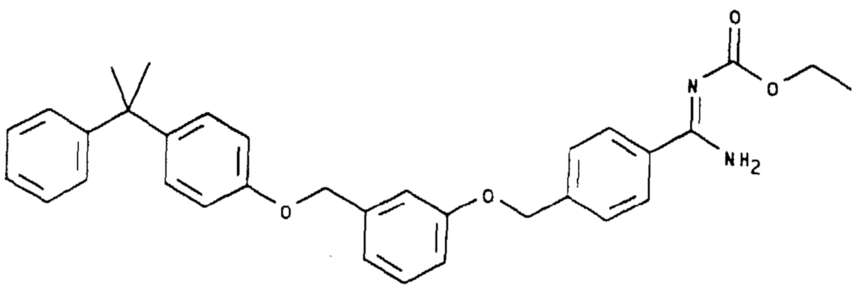

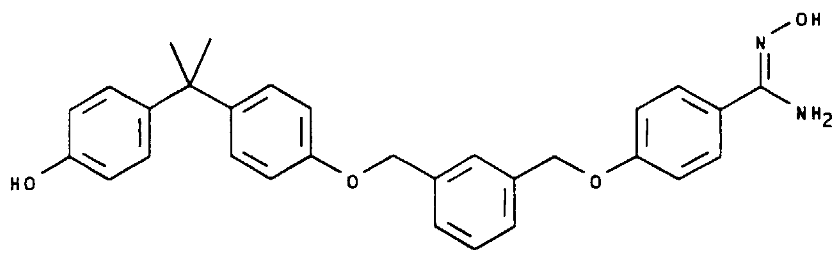

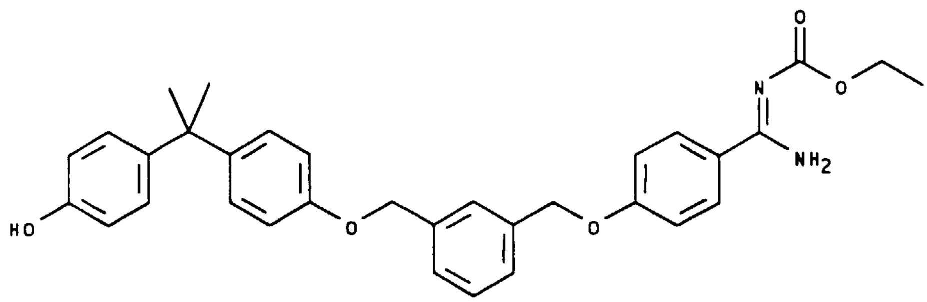

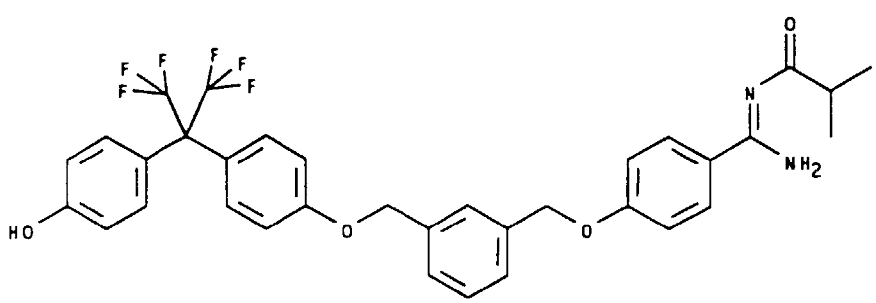

## Patentansprüche

1. Neue substituierte Benzamidine der Formel

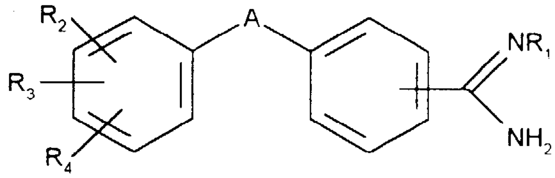

(I)

in der

A für die Gruppe

$$-X_1-C_mH_{2m}-X_2- \qquad (II)$$

(m gleich 2 bis 6)
oder

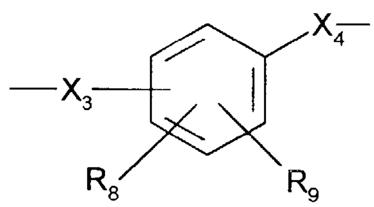

(III)

steht:

$X_1$ für O, NH oder $NCH_3$

$X_2$ für O, NH, $NCH_3$ oder

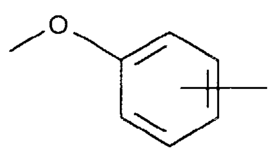

$X_3$ für $X-C_nH_{2n}-$;

$X_4$ für $-C_nH_{2n}-X-$ (n = 1 oder 2, X = O, NH oder $NCH_3$);

$R_1$ für OH, CN, $COR_{12}$, $COOR_{12}$ und CHO;

$R_2$ für Br, Cl, F, $CF_3$, OH, $C_1$-$C_6$-Alkyl, $C_5$-$C_7$-Cycloalkyl, Aryl, O-Aryl, $CH_2$-Aryl, $CR_5R_6$-Aryl, $C(CH_3)_2$-$R_7$;
außerdem für H, wenn A ein Rest der Formel III oder wenn $X_2$ ein Rest der Formel IV ist;
außerdem für $C_1$-$C_6$-Alkoxy, wenn A die Gruppe II ist, $X_1$ und m die obige Bedeutung haben und $X_2$ für NH, $NCH_3$ oder die Gruppe IV steht,

oder wenn A die Gruppe III bedeutet, $X_3$ die obige Bedeutung hat und in $X_4$ X für NH oder $NCH_3$ steht;

$R_3$ für H, $C_1$-$C_6$-Alkyl, OH, Cl, F, außerdem für $C_1$-$C_6$-Alkoxy, wenn $R_2$ Aryl, O-Aryl, $CH_2$-Aryl, $CR_5R_6$-Aryl oder $C(CH_3)_2$-$R_7$ ist oder wenn $X_2$ die Gruppe IV bedeutet;
$R_2$ und $R_3$ können gemeinsam auch einen ankondensierten aromatischen oder heteroaromatischen Ring bedeuten;

$R_4$ für H oder $C_1$-$C_6$-Alkyl;

$R_5$ für $C_1$-$C_4$-Alkyl, $CF_3$, $CH_2OH$, COOH oder COO($C_1$-$C_4$-Alkyl);

$R_6$ für H, $C_1$-$C_4$-Alkyl oder $CF_3$;
$R_5$ und $R_6$ können gemeinsam auch eine $C_4$-$C_6$-Alkylengruppe bilden;

$R_7$ für $CH_2OH$, COOH, COO($C_1$-$C_4$-Alkyl), $CONR_{10}R_{11}$ oder $CH_2NR_{10}R_{11}$;

$R_8$, $R_9$ für H, Br, Cl, F, OH, $C_1$-$C_6$-Alkyl oder $C_1$-$C_6$-Alkoxy;

$R_{10}$ für H, $C_1$-$C_6$-Alkyl, Phenyl, Phenyl-($C_1$-$C_6$-Alkyl), $COR_{12}$, $COOR_{12}$, CHO, $CONH_2$, $CONHR_{12}$, $SO_2$-($C_1$-$C_6$-Alkyl), $SO_2$-Phenyl,
wobei der Phenylrest ein- oder mehrfach durch Cl, F, $CF_3$, $C_1$-$C_4$-Alkyl, OH, $C_1$-$C_4$-Alkoxy substituiert sein kann;

$R_{11}$ H oder $C_1$-$C_6$-Alkyl;
$R_{10}$ und $R_{11}$ können gemeinsam auch eine $C_4$-$C_6$-Alkylengruppe darstellen;

$R_{12}$ für $C_1$-$C_6$-Alkyl, $C_5$-$C_7$-Cycloalkyl, Aryl, Heteroaryl, Aralkyl oder Heteroaryl-($C_1$-$C_6$-Alkyl), wobei die Aryl- oder Heteroarylgruppen ein- oder mehrfach durch Cl, F, $CF_3$, $C_1$-$C_4$-Alkyl, OH oder $C_1$-$C_4$-Alkoxy substituiert sein können

mit der Maßgabe, daß im Falle

A für eine Gruppe -$X_1$-$C_mH_{2m}$-$X_2$- steht,
worin m eine ganze Zahl 2,3 oder 4 und

$X_1$ O, NH,

$X_2$ O, NH oder

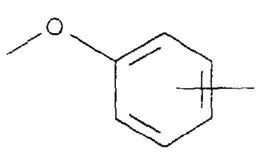

und

$R_2$ Wasserstoff, Br, Cl, F, $CF_3$, $C_1$-$C_6$-Alkyl, Phenyl,

$R_3$ Wasserstoff, $C_1$-$C_6$-Alkyl, Hydroxy, Cl, F, $C_1$-$C_6$-Alkoxy,

$R_4$ Wasserstoff, $C_1$-$C_6$-Alkyl

bedeuten,

$R_1$ nicht die Bedeutung von -OH haben darf.

2. Verbindungen nach Anspruch 1, in denen Aryl Phenyl oder ein- oder mehrfach substituiertes Phenyl und Heteroaryl Pyridyl, Pyridazinyl, Pyrimidyl, Pyrazinyl, Thienyl oder Furyl bedeutet.

3. Verbindungen nach Anspruch 1 oder 2, worin

| | |
|---|---|
| A, m, n, $X_3$, $X_4$ und $R_1$ | die obige Bedeutung haben; |
| $X_1$ | für O; |
| $X_2$ | für O oder eine Gruppe IV ( worin $X_1$ gleich O ist); |
| $R_2$ | für Cl, F, $CF_3$, OH, $C_1$-$C_6$-Alkyl, Aryl, O-Aryl, $CH_2$-Aryl oder $CR_5R_6$-Aryl, und, wenn $X_2$ die Gruppe IV ist außerdem für $C_1$-$C_6$-Alkoxy; |
| $R_3$ | für H, $C_1$-$C_6$-Alkyl oder OH, und, wenn $R_2$ $CR_5R_6$-Aryl ist, außerdem für $C_1$-$C_6$-Alkoxy; |
| $R_4$ | für H; |
| $R_5$ | für $C_1$-$C_3$-Alkyl, $CF_3$ oder $CH_2OH$; |
| $R_6$ | für H, $C_1$-$C_3$-Alkyl oder $CF_3$, |
| $R_5$ und $R_6$ | gemeinsam auch für $C_4$-$C_5$-Alkylen; |
| $R_8$, $R_9$ | für H, F oder OH |

stehen.

4. Die Verbindungen nach Anspruch 1 oder 2, worin

| | |
|---|---|
| $X_1$ | für O; |
| $X_2$ | für die Gruppe IV (worin $X_1$ gleich O ist); |
| X | für O; |
| $R_1$ | für $COOR_{12}$; |
| $R_2$ | für $C_1$-$C_6$-Alkyl, Aryl, O-Aryl, $CH_2$-Aryl oder $CR_5R_6$-Aryl; |
| $R_3$ | für H, OH oder $C_1$-$C_6$-Alkyl und, wenn $R_2$ $CR_5R_6$-Aryl ist, auch für $C_1$-$C_6$-Alkoxy; |
| $R_4$ | für H; |
| $R_5$, $R_6$ | für $C_1$-$C_3$-Alkyl oder $CF_3$; |
| $R_8$,$R_9$ | für H; |
| $R_{12}$ | für $C_1$-$C_6$-Alkyl, Aralkyl oder $C_7$-$C_5$-Cycloalkyl |

stehen

5. Methoxycarbonyl-imino-{4'-[2-(2-propylphenoxy)-ethoxy]-biphenyl-4-yl}-methyl)-amin und seine Säureadditionssalze.

6. (Benzyloxycarbonyl-imino-{4'-[2-(2-propylphenoxy)-ethoxy]-biphenyl-4-yl}methyl)-amin und seine Säureadditionssalze.

7. [Hydroxy-imino-(4-{3-[4-(1-methyl-1-phenylethyl)-phenoxymethyl]-benzyloxy}phenyl)-methyl]-amin und seine Säureadditionssalze

8. [Ethoxycarbonyl-imino-(4-{3-[4-(1-methyl-1-phenylethyl)-phenoxymethyl]-benzyloxy}phenyl)-methyl]-amin und seine Säureadditionssalze.

9. [3'-Pyridylcarbonyl-imino-(4-{3-[4-(1 -methyl-1-phenylethyl)-phenoxymethyl]-benzyloxy}phenyl)-methyl]-amin und seine Säureadditionssalze.

10. Pharmazeutische Zubereitungen mit üblichen Hilfs- und/oder Trägerstoffen, gekennzeichnet durch einen Gehalt an einer Verbindung nach Anspruch 1 bis 9.

11. Verwendung von Verbindungen der allgemeinen Formel I

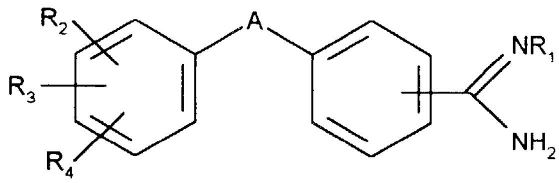

(I)

in der

A für die Gruppe

$$-X_1-C_mH_{2m}-X_2- \quad (II)$$

(m gleich 2 bis 6)
oder

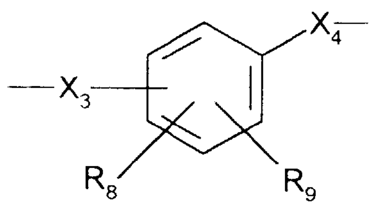

(III)

steht:

$X_1$ für O, NH oder $NCH_3$

$X_2$ für O, NH, $NCH_3$ oder

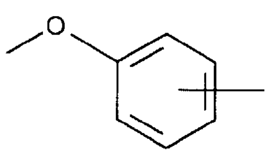

$X_3$ für $-X-C_nH_{2n}-$;

$X_4$ für $-C_nH_{2n}-X-$ (n = 1 oder 2, X = O, NH oder $NCH_3$);

$R_1$ für OH, CN, $COR_{12}$, $COOR_{12}$ und CHO;

$R_2$ für Br, Cl, F, $CF_3$, OH, $C_1$-$C_6$-Alkyl, $C_5$-$C_7$-Cycloalkyl, Aryl, O-Aryl, $CH_2$-Aryl, $CR_5R_6$-Aryl, $C(CH_3)_2$-$R_7$;
außerdem für H, wenn A ein Rest der Formel III oder wenn $X_2$ ein Rest der Formel IV ist;
außerdem für $C_1$-$C_6$-Alkoxy, wenn A die Gruppe II ist, $X_1$ und m die obige Bedeutung haben und $X_2$ für NH, $NCH_3$ oder die Gruppe IV steht,
oder wenn A die Gruppe III bedeutet, $X_3$ die obige Bedeutung hat und in $X_4$ X für NH oder $NCH_3$ steht;

$R_3$ für H, $C_1$-$C_6$-Alkyl, OH, Cl, F, außerdem für $C_1$-$C_6$-Alkoxy, wenn $R_2$ Aryl, O-Aryl, $CH_2$-Aryl, $CR_5R_6$-Aryl oder $C(CH_3)_2$-$R_7$ ist oder wenn $X_2$ die Gruppe IV bedeutet;

$R_2$ und $R_3$ können gemeinsam auch einen ankondensierten aromatischen oder heteroaromatischen Ring bedeuten;

$R_4$ für H oder $C_1$-$C_6$-Alkyl;

$R_5$ für $C_1$-$C_4$-Alkyl, $CF_3$, $CH_2OH$, COOH oder COO($C_1$-$C_4$-Alkyl);

$R_6$ für H, $C_1$-$C_4$-Alkyl oder $CF_3$;

$R_5$ und $R_6$ können gemeinsam auch eine $C_4$-$C_6$-Alkylengruppe bilden;

$R_7$ für $CH_2OH$, COOH, COO($C_1$-$C_4$-Alkyl), $CONR_{10}R_{11}$ oder $CH_2NR_{10}R_{11}$;

$R_8$, $R_9$ für H, Br, Cl, F, OH, $C_1$-$C_6$-Alkyl oder $C_1$-$C_6$-Alkoxy;

$R_{10}$ für H, $C_1$-$C_6$-Alkyl, Phenyl, Phenyl-($C_1$-$C_6$-Alkyl), $COR_{12}$, $COOR_{12}$, CHO, $CONH_2$, $CONHR_{12}$, $SO_2$-($C_1$-$C_6$-Alkyl), $SO_2$-Phenyl, wobei der Phenylrest ein- oder mehrfach durch Cl, F, $CF_3$, $C_1$-$C_4$-Alkyl, OH, $C_1$-$C_4$-Alkoxy substituiert sein kann;

$R_{11}$ H oder $C_1$-$C_6$-Alkyl;

$R_{10}$ und $R_{11}$ können gemeinsam auch eine $C_4$-$C_6$-Alkylengruppe darstellen;

$R_{12}$ für $C_1$-$C_6$-Alkyl, $C_5$-$C_7$-Cycloalkyl, Aryl, Heteroaryl, Aralkyl oder Heteroaryl-($C_1$-$C_6$-Alkyl), wobei die Aryl- oder Heteroarylgruppen ein- oder mehrfach durch Cl, F, $CF_3$, $C_1$-$C_4$-Alkyl, OH oder $C_1$-$C_4$-Alkoxy substituiert sein können;

bei der Herstellung von Arzneimitteln für die Behandlung von Krankheiten, bei denen $LBT_4$ -Antagonisten therapeutisch angewendet werden können.

**12.** Verwendung von Verbindungen gemäß Anspruch 11 zur Herstellung eines Arzneimittels für die Behandlung von Krankheiten, bei denen entzündliche und/oder allergische Vorgänge eine Rolle spielen, insbesondere Arthritis, Asthma, chronischen obstruktiven Lungenerkrankungen, Psoriasis, Colitis ulcerosa, Alzheimer-Krankheit, Schock, Atherosklerose, Multiple Sklerose, und zur Behandlung einer durch nichtsteroidale Antiphlogistika induzierten Gastropathie sowie bei Metastasis und chronischer myelozytischer Leukämie.

**13.** Verfahren zur Herstellung der Verbindungen nach Anspruch 1 bis 9, dadurch gekennzeichnet, daß man

a.) ein Amidin der Formel

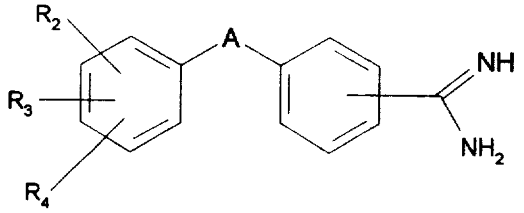

(V)

mit einer Verbindung der Formel

$$L - R'_1 \qquad (VI)$$

wobei in (V) A, $R_2$, $R_3$ und $R_4$ die obige Bedeutung haben, $R'_1$ dieselbe Bedeutung wie $R_1$ hat, ausgenommen OH, und L eine nucleophile Austrittsgruppe wie ein Halogenatom (etwa Cl, Br) oder Acyloxy darstellt, umsetzt, oder, daß man

b.) eine Verbindung der Formel

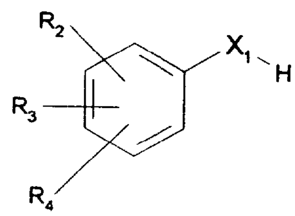

(VII)

(worin $R_2$, $R_3$, $R_4$ und $X_1$ die obige Bedeutung haben), mit einem Benzamidinderivat der Formel

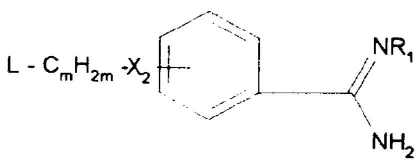

(VIII)

oder

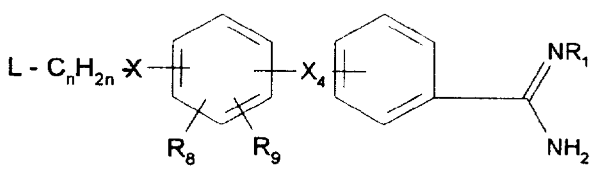

(IX)

(worin L, m, n, X, $X_2$, $X_4$, $R_1$, $R_8$ und $R_9$ die obige Bedeutung haben), umsetzt,
oder daß man

c.) eine Verbindung der Formel

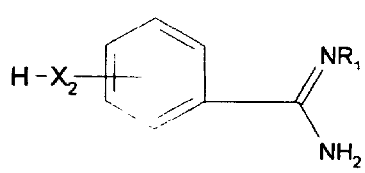

(X)

($R_1$ und $X_2$ in der obigen Bedeutung) mit einer Verbindung der Formel

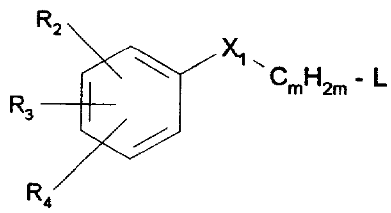

(XI)

oder

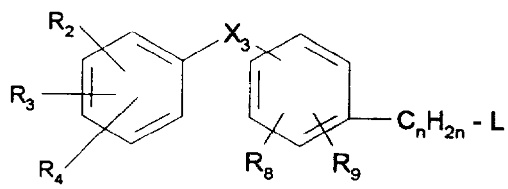

(XII)

(worin L, m, n, $R_2$, $R_3$, $R_4$, $R_8$, $R_9$, $X_1$ und $X_3$ die obige Bedeutung haben) umsetzt, oder, daß man

d.) zur Herstellung solcher Verbindungen der Formel I, in denen $R_1$ OH bedeutet, ein Nitril der Formel

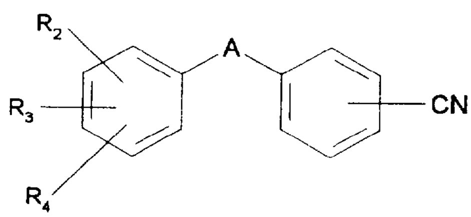

(XIII),

worin A, $R_2$, $R_3$, und $R_4$ die obige Bedeutung haben, mit Hydroxylamin umsetzt und aus den erhaltenen Produkten, je nach ihrer Art, gegebenenfalls optische Antipoden oder diastereomere Antipodenpaare herstellt oder daß man aus erhaltenen Basen Säureadditionssalze, aus zunächst erhaltenen Säureadditionssalzen freie Basen gewinnt.

## Claims

**1.** New substituted benzamidines of formula

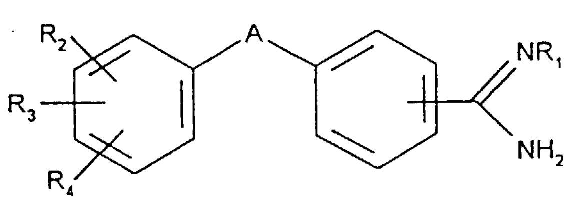

(I)

wherein

A denotes the group

$$-X_1-C_mH_{2m}-X_2- \quad (II)$$

(m equals 2 to 6)

or

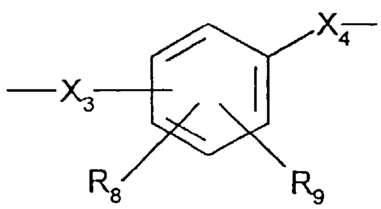

(III);

$X_1$ denotes O, NH or $NCH_3$

$X_2$ denotes O, NH, $NCH_3$ or

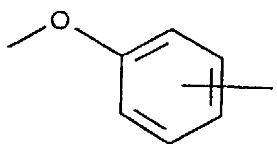

(IV)

$X_3$ denotes $-X-C_nH_{2n}-$;

$X_4$ denotes $-C_nH_{2n}-X-$ (n = 1 or 2, X = O, NH or $NCH_3$);

$R_1$ denotes OH, CN, $COR_{12}$, $COOR_{12}$ or CHO;

$R_2$ denotes Br, Cl, F, $CF_3$, OH, $C_1$-$C_6$-alkyl, $C_5$-$C_7$-cycloalkyl, aryl, O-aryl, $CH_2$-aryl, $CR_5R_6$-aryl, $C(CH_3)_2$-$R_7$;
and also H, when A is a group of Formula III or when $X_2$ is a group of Formula IV;
and also $C_1$-$C_6$-alkoxy, when A is the group II, $X_1$ and m are as hereinbefore defined and $X_2$ denotes NH, $NCH_3$ or the group IV,
or when A denotes the group III, $X_3$ is as hereinbefore defined and in $X_4$ X denotes NH or $NCH_3$;

$R_3$ denotes H, $C_1$-$C_6$-alkyl, OH, Cl, F, and also $C_1$-$C_6$ alkoxy when $R_2$ denotes aryl, O-aryl, $CH_2$-aryl, $CR_5R_6$-aryl or $C(CH_3)_2$-$R_7$ or when $X_2$ denotes the group IV;

$R_2$ and $R_3$ may also together denote a fused aromatic or heteroaromatic ring;

$R_4$ denotes H or $C_1$-$C_6$-alkyl;

$R_5$ denotes $C_1$-$C_4$-alkyl, $CF_3$, $CH_2OH$, COOH or COO($C_1$-$C_4$-alkyl);

$R_6$ denotes H, $C_1$-$C_4$-alkyl, or $CF_3$;

$R_5$ and $R_6$ together may also form a $C_4$-$C_6$-alkylene group;

$R_7$ denotes $CH_2OH$, COOH, COO($C_1$-$C_4$-alkyl), $CONR_{10}R_{11}$ or $CH_2NR_{10}R_{11}$;

$R_8$, $R_9$ denote H, Br, Cl, F, OH, $C_1$-$C_6$-alkyl or $C_1$-$C_6$-alkoxy;

$R_{10}$ denotes H, $C_1$-$C_6$-alkyl, phenyl, phenyl-($C_1$-$C_6$-alkyl), $COR_{12}$, $COOR_{12}$, CHO, $CONH_2$, $CONHR_{12}$, $SO_2$-($C_1$-$C_6$-alkyl), $SO_2$-phenyl,
wherein the phenyl group may be mono- or polysubstituted by Cl, F, $CF_3$, $C_1$-$C_4$-alkyl, OH and/or $C_1$-$C_4$-alkoxy;

$R_{11}$ denotes H or $C_1$-$C_6$-alkyl;

$R_{10}$ and $R_{11}$ together may also denote a $C_4$-$C_6$-alkylene group;

$R_{12}$ denotes $C_1$-$C_6$-alkyl, $C_5$-$C_7$-cycloalkyl, aryl, heteroaryl, aralkyl or heteroaryl-($C_1$-$C_6$-alkyl), wherein the aryl or heteroaryl group may be mono or polysubstituted by Ci, F, $CF_3$, $C_1$-$C_4$-alkyl, OH or $C_1$-$C_4$-alkoxy,

with the proviso that if

A denotes a group $-X_1-C_mH_{2m}-X_2-$
wherein m represents an integer 2, 3, or 4; and

$X_1$ denotes O, NH;

$X_2$ denotes O, NH or

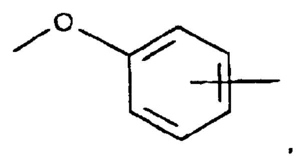

and

$R_2$ denotes hydrogen, Br, Cl, $CF_3$, $C_1$-$C_6$-alkyl, phenyl;

$R_3$ denotes hydrogen, $C_1$-$C_6$-alkyl, hydroxy, Cl, F, $C_1$-$C_6$-alkoxy;

$R_4$ denotes hydrogen, $C_1$-$C_6$-alkyl;

$R_1$ must not denote hydroxy.

**2.** Compounds as defined in claim 1, wherein aryl denotes phenyl or mono- or polysubstituted phenyl and heteroaryl denotes pyridyl, pyridazinyl, pyrimidyl, pyrazinyl, thienyl or furyl.

**3.** Compounds as defined in claim 1 or 2, wherein

| | |
|---|---|
| A, m, n, $X_3$, $X_4$ and $R_1$ | are as hereinbefore defined; |
| $X_1$ | denotes O; |
| $X_2$ | denotes O or a group IV (wherein $X_1$ equals O); |
| $R_2$ | denotes Cl, F, $CF_3$, OH, $C_1$-$C_6$-alkyl, aryl, O-aryl, $CH_2$-aryl or $CR_5R_6$-aryl, and, if $X_2$ is the group IV, $R_2$ may also represent $C_1$-$C_6$-alkoxy; |
| $R_3$ | denotes H, $C_1$-$C_6$-alkyl or OH, and, if $R_2$ is $CR_5R_6$-aryl, $R_3$ may also denote $C_1$-$C_6$-alkoxy; |
| $R_4$ | denotes H; |
| $R_5$ | denotes $C_1$-$C_3$-alkyl, $CF_3$ or $CH_2OH$; |
| $R_6$ | denotes H, $C_1$-$C_3$-alkyl or $CF_3$, |
| $R_5$ and $R_6$ | together may also denote $C_4$-$C_5$-alkylene; |
| $R_8$ and $R_9$ | denote H, F or OH. |

**4.** Compounds as defined in claim 1 or 2 wherein

| | |
|---|---|
| $X_1$ | denotes O; |
| $X_2$ | represents the group IV (wherein $X_1$ equals O); |
| X | denotes O; |
| $R_1$ | denotes $COOR_{12}$; |
| $R_2$ | denotes $C_1$-$C_6$-alkyl, aryl, O-aryl, $CH_2$-aryl or $CR_5R_6$-aryl; |
| $R_3$ | denotes H, OH or $C_1$-$C_6$-alkyl and, if $R_2$ is $CR_5R_6$-aryl, $R_3$ may also denote $C_1$-$C_6$ alkoxy; |
| $R_4$ | denotes H; |
| $R_5$ and $R_6$ | denote $C_1$-$C_3$-alkyl or $CF_3$; |
| $R_8$ and $R_9$ | denote H; |
| $R_{12}$ | denotes $C_1$-$C_6$-alkyl, aralkyl or $C_3$-$C_5$-cycloalkyl. |

5. (Methoxycarbonyl-imino-{4'-[2-(2-propylphenoxy)-ethoxy]-biphenyl-4-yl}-methyl)-amine and the acid addition salts thereof.

6. (Benzyloxycarbonyl-imino-{4'-[2-(2-propylphenoxy)-ethoxy]-biphenyl-4-yl}-methyl)-amine and the acid addition salts thereof.

7. [Hydroxy-imino-(4-{3-[4-(1-methyl-1-phenylethyl)-phenoxymethyl]-benzyloxy}phenyl)-methyl]-amine and the acid addition salts thereof.

8. [Ethoxycarbonyl-imino-(4-{3-[4-(1-methyl-1-phenylethyl)-phenoxymethyl]-benzyloxy}phenyl)-methyl]-amine and the acid addition salts thereof.

9. [3'-Pyridylcarbonyl-imino-(4-{3-[4-(1-methyl-1-phenylethyl)-phenoxymethyl]-benzyloxy)phenyl)-methyl]-amine and the acid addition salts thereof.

10. Pharmaceutical preparations characterised in that they contain a compound with conventional excipients and/or carriers, according to claims 1 to 9.

11. Use of compounds of formula

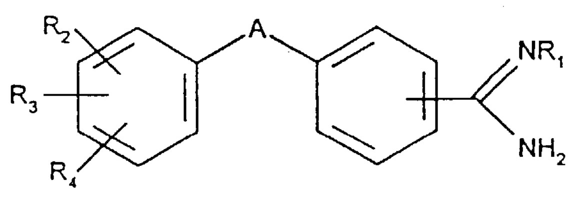

(I)

wherein

A denotes the group

$$-X_1-C_mH_{2m}-X_2- \quad (II)$$

(m equals 2 to 6)

or

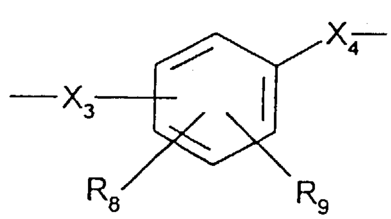

(III);

$X_1$ denotes O, NH or $NCH_3$

$X_2$ denotes O, NH, $NCH_3$ or

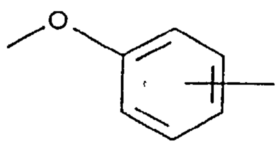

(IV)

| | |
|---|---|
| $X_3$ | denotes $-X-C_nH_{2n}-$; |
| $X_4$ | denotes $-C_nH_{2n}-X-$ (n = 1, or 2, X = O NH or $NCH_3$); |
| $R_1$ | denotes OH, CN, $COR_{12}$, $COOR_{12}$ or CHO; |
| $R_2$ | denotes Br, Cl, F, $CF_3$, OH, $C_1$-$C_6$-alkyl, $C_5$-$C_7$-cycloalkyl, aryl, O-aryl, $CH_2$-aryl, $CR_5R_6$-aryl, $C(CH_3)_2$-$R_7$; and also H, when A is a group of Formula III or when $X_2$ is a group of Formula IV; and also $C_1$-$C_6$-alkoxy, when A is the group II, $X_1$ and m are as hereinbefore defined and $X_2$ denotes NH, $NCH_3$ or the group IV, or when A denotes the group III, $X_3$ is as hereinbefore defined and in $X_4$ X denotes NH or $NCH_3$; |
| $R_3$ | denotes H, $C_1$-$C_6$-alkyl, OH, Cl, F, and also $C_1$-$C_6$ alkoxy when $R_2$ denotes aryl, O-aryl, $CH_2$-aryl, $CR_5R_6$-aryl or $C(CH_3)_2$-$R_7$ or when $X_2$ denotes the group IV; |
| $R_2$ and $R_3$ | may also together denote a fused aromatic or heteroaromatic ring; |
| $R_4$ | denotes H or $C_1$-$C_6$-alkyl; |
| $R_5$ | denotes $C_1$-$C_4$-alkyl, $CF_3$, $CH_2OH$, COOH or COO($C_1$-$C_4$-alkyl); |
| $R_6$ | denotes H, $C_1$-$C_4$-alkyl, or $CF_3$; |
| $R_5$ and $R_6$ | together may also form a $C_4$-$C_6$-alkylene group; |
| $R_7$ | denotes $CH_2OH$, COOH, COO($C_1$-$C_4$-alkyl), $CONR_{10}R_{11}$ or $CH_2NR_{10}R_{11}$; |
| $R_8$, $R_9$ | denote H, Br, Cl, F, OH, $C_1$-$C_6$-alkyl or $C_1$-$C_6$-alkoxy; |
| $R_{10}$ | denotes H, $C_1$-$C_6$-alkyl, phenyl, phenyl-($C_1$-$C_6$-alkyl), $COR_{12}$, $COOR_{12}$, CHO, $CONH_2$, $CONHR_{12}$, $SO_2$-($C_1$-$C_6$-alkyl), $SO_2$-phenyl, wherein the phenyl group may be mono- or polysubstituted by Cl, F, $CF_3$, $C_1$-$C_4$-alkyl, OH and/or $C_1$-$C_4$-alkoxy; |
| $R_{11}$ | denotes H or $C_1$-$C_6$-alkyl; |
| $R_{10}$ and $R_{11}$ | together may also denote a $C_4$-$C_6$-alkylene group; |
| $R_{12}$ | denotes $C_1$-$C_6$-alkyl, $C_5$-$C_7$-cycloalkyl, aryl, heteroaryl, aralkyl or heteroaryl-($C_1$-$C_6$-alkyl), wherein the aryl or heteroaryl group may be mono or polysubstituted by Cl, F, $CF_3$, $C_1$-$C_4$-alkyl, OH or $C_1$-$C_4$-alkoxy; |

in the preparation of pharmaceutical compositions for treating diseases in which $LBT_4$-antagonists can be used therapeutically.

**12.** Use of compounds as defined claim 11 for preparing a pharmaceutical composition for the treatment of diseases in which inflammatory and/or allergic processes play a part, especially arthritis, asthma, chronic obstructive lung diseases, psoriasis, ulcerative colitis, Alzheimer's disease, shock, atherosclerosis, multiple sclerosis, and for the treatment of gastropathy induced by non-steroidal antiphlogistics and in metastasis and chronic myelocytic leukaemia.

**13.** A process for preparing the compounds as defined in any one of claims 1 to 9, characterised in that

a.) an amidine of the formula

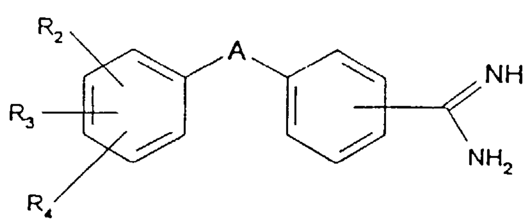

(V)

is reacted with a compound of formula

$$L - R'_1 \qquad (VI)$$

wherein in (V) A, $R_2$, $R_3$ and $R_4$ are as hereinbefore defined, $R'_1$ has the same meaning as $R_1$, with the exception of OH, and L denotes a nucleophilically exchangeable group such as a halogen atom (e.g. Cl, Br) or acyloxy,
or

b.) a compound of formula

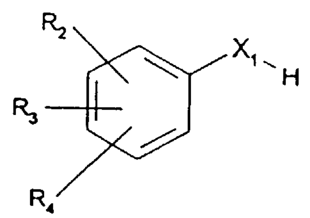

(VII)

(wherein $R_2$, $R_3$, $R_4$ and $X_1$ are as hereinbefore defined) is reacted with a benzamidine derivative of the formula

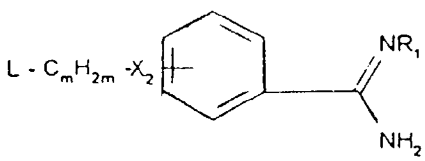

(VIII)

or

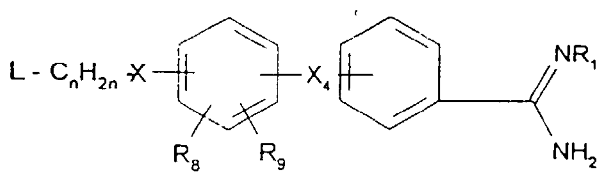

(IX)

(wherein L, m, n, X, $X_2$, $X_4$, $R_1$, $R_8$ and $R_9$ are as hereinbefore defined),
or

c.) a compound of formula

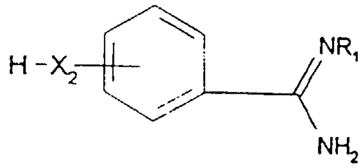

(X)

(wherein $R_1$ and $X_2$ are as hereinbefore defined) is reacted with a compound of formula

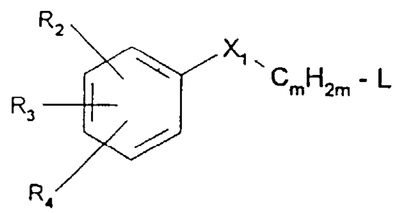

(XI)

or

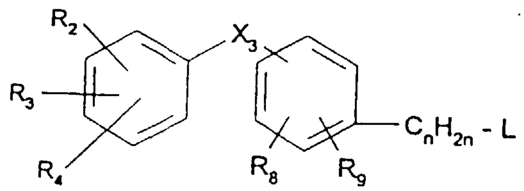

(XII)

(wherein L, m, n, $R_2$, $R_3$, $R_4$, $R_8$, $R_9$, $X_1$ and $X_3$ are as hereinbefore defined), or

d.) in order to prepare compounds of formula I wherein $R_1$ denotes OH, a nitrile of formula

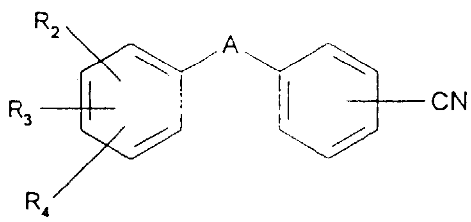

(XIII),

wherein A, $R_2$, $R_3$ and $R_4$ are as hereinbefore defined, is reacted with hydroxylamine

and from the resulting products, depending on their nature, optical isomers or diastereomeric pairs of isomers may be prepared or, if bases are obtained, acid addition salts may be recovered and if acid addition salts are obtained initially, free bases may be recovered.

## Revendications

**1.** Nouvelles benzamidines substituées de formule

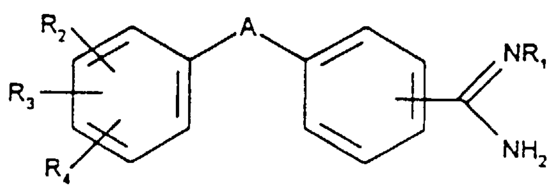

(I)

dans laquelle

A représente le groupe

$-X_1-C_mH_{2m}-X_2-$ (II)

(m est égal à un nombre de 2 à 6)

ou

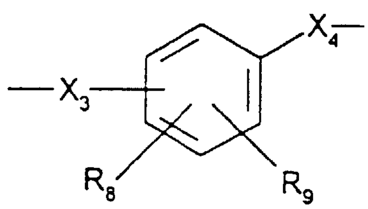

(III);

$X_1$ représente O, NH ou $NCH_3$,

$X_2$ représente O, NH, $NCH_3$ ou

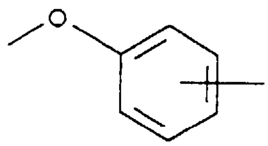

$X_3$ est $-X-C_nH_{2n}-$;

$X_4$ est $-C_nH_{2n}-X-$(n = 1 ou 2, X = O, NH ou $NCH_3$);

$R_1$ est OH, CN, $COR_{12}$, $COOR_{12}$ et CHO;

$R_2$ représente Br, Cl, F, $CF_3$, OH, alkyle en $C_1$-$C_6$, cycloalkyle en $C_5$-$C_7$, aryle, O-aryle, $CH_2$-aryle, $CR_5R_6$-aryle, $C(CH_3)_2-R_7$, et aussi H, lorsque A est un reste de formule III ou lorsque $X_2$ est un reste de formule IV, et aussi alcoxy en $C_1$-$C_6$, lorsque A est le groupe II, $X_1$ et m ont la signification indiquée ci-dessus et $X_2$ représente NH, $NCH_3$ ou le groupe IV, ou lorsque A représente le groupe III, $X_3$ a la signification indiquée ci-dessus et, dans $X_4$, X est NH ou $NCH_3$;

$R_3$ représente H, alkyle en $C_1$-$C_6$, OH, Cl, F, et aussi alcoxy en $C_1$-$C_6$ lorsque $R_2$ est aryle, O-aryle, $CH_2$-aryle, $CR_5R_6$-aryle ou $C(CH_3)_2-R_7$ ou lorsque $X_2$ représente le groupe IV;

$R_2$ et $R_3$ peuvent aussi représenter ensemble un noyau aromatique ou hétéroaromatique condensé;

$R_4$ est H ou alkyle en $C_1$-$C_6$;

$R_5$ est alkyle en $C_1$-$C_4$, $CF_3$, $CH_2OH$, COOH ou COO(alkyle en $C_1$-$C_4$);

$R_6$ est H, alkyle en $C_1$-$C_4$ ou $CF_3$;

$R_5$ et $R_6$ peuvent aussi former ensemble un groupe alkylène en $C_4$-$C_6$;

$R_7$ est $CH_2OH$, COOH, COO(alkyle en $C_1$-$C_4$), $CONR_{10}R_{11}$ ou $CH_2NR_{10}R_{11}$;

$R_8$, $R_9$ sont H, Br, Cl, F, OH, alkyle en $C_1$-$C_6$ ou alcoxy en $C_1$-$C_6$;

$R_{10}$ est H, alkyle en $C_1$-$C_6$, phényle, phényl(alkyle en $C_1$-$C_6$), $COR_{12}$, $COOR_{12}$, CHO, $CONH_2$, $CONHR_{12}$, $SO_2$-(alkyle en $C_1$-$C_6$), $SO_2$-phényle, le reste phényle pouvant être substitué une ou plusieurs fois par Cl, F, $CF_3$, alkyle en $C_1$-$C_4$, OH, alcoxy en $C_1$-$C_4$;

$R_{11}$ est H ou alkyle en $C_1$-$C_6$;

$R_{10}$ et $R_{11}$ peuvent aussi représenter ensemble un groupe alkylène en $C_4$-$C_6$;

$R_{12}$ est alkyle en $C_1$-$C_6$, cycloalkyle en $C_5$-$C_7$, aryle, hétéroaryle, aralkyle ou hétéroaryl(alkyle en $C_1$-$C_6$), les groupes aryle ou hétéroaryle pouvant être substitués une ou plusieurs fois par Cl, F, $CF_3$, alkyle en $C_1$-$C_4$, OH ou alcoxy en $C_1$-$C_4$,

à condition que $R_1$ n'ait pas la signification de -OH dans le cas où

A représente un groupe $-X_1-C_mH_{2m}-X_2-$ dans lequel m est un nombre entier égal à 2, 3 ou 4 et

$X_1$ est O, NH,
$X_2$ est O, NH ou

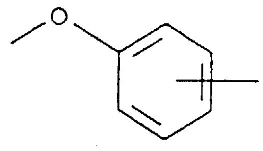

et
$R_2$ est H, Br, Cl, F, $CF_3$, alkyle en $C_1$-$C_6$, phényle,
$R_3$ est H, alkyle en $C_1$-$C_6$, hydroxy, Cl, F, alcoxy en $C_1$-$C_6$,
$R_4$ est H, alkyle en $C_1$-$C_6$.

**2.** Composés selon la revendication 1, dans lesquels aryle désigne un groupe phényle ou phényle substitué une ou plusieurs fois, et hétéroaryle désigne un groupe pyridyle, pyridazinyle, pyrimidyle, pyrazinyle, thiényle ou furyle.

**3.** Composés selon la revendication 1 ou 2, dans lesquels

| | |
|---|---|
| A, m, n, $X_3$, $X_4$ et $R_1$ | ont la signification indiquée ci-dessus; |
| $X_1$ | est O; |
| $X_2$ | est O ou un groupe IV (dans lequel $X_1$ est O); |
| $R_2$ | est Cl, F, $CF_3$, OH, alkyle en $C_1$-$C_6$, aryle, O-aryle, $CH_2$-aryle ou $CR_5R_6$-aryle, et, lorsque $X_2$ est le groupe IV, $R_2$ est en outre alcoxy en $C_1$-$C_6$; |
| $R_3$ | est H, alkyle en $C_1$-$C_6$ ou OH, et, lorsque $R_2$ est $CR_5R_6$-aryle, $R_3$ est en outre alcoxy en $C_1$-$C_6$; |
| $R_4$ | est H; |
| $R_5$ | est alkyle en $C_1$-$C_3$, $CF_3$ ou $CH_2OH$; |
| $R_6$ | est H, alkyle en $C_1$-$C_3$ ou $CF_3$; |
| $R_5$ et $R_6$ | sont aussi ensemble un alkylène en $C_4$-$C_5$; |
| $R_8$, $R_9$ | sont H, F ou OH. |

**4.** Composés selon la revendication 1 ou 2, dans lesquels

| | |
|---|---|
| $X_1$ | est O; |
| $X_2$ | représente le groupe IV (dans lequel $X_1$ est O); |
| X | est O; |
| $R_1$ | est $COOR_{12}$; |
| $R_2$ | est alkyle en $C_1$-$C_6$, aryle, O-aryle, $CH_2$-aryle ou $CR_5R_6$-aryle; |
| $R_3$ | est H, OH ou alkyle en $C_1$-$C_6$, et, lorsque $R_2$ est $CR_5R_6$-aryle, $R_3$ est aussi alcoxy en $C_1$-$C_6$; |
| $R_4$ | est H; |
| $R_5$, $R_6$ | sont alkyle en $C_1$-$C_3$ ou $CF_3$; |
| $R_8$, $R_9$ | sont H; |
| $R_{12}$ | est alkyle en $C_1$-$C_6$, aralkyle ou cycloalkyle en $C_5$-$C_7$. |

**5.** (Méthoxycarbonylîmino-{4,-[2-(2-propylphénoxy)éthoxy]biphényl-4-yl}méthyl)amine et ses sels d'addition d'acides.

**6.** (Benzyloxycarbonylimino-{4'-[2-(2-propylphénoxy)éthoxy]biphényl-4-yl}méthyl)amine et ses sels d'addition d'acides.

**7.** [Hydroxyimino-(4-{3-[4-(1-méthyl-1 -phényléthyl)phénoxyméthyl]benzyloxy}phényl)méthyl]amine et ses sels d'addition d'acides.

**8.** [Éthoxycarbonylimino-(4-{3-[4-(1-méthyl-1-phényléthyl)phénoxyméthyl]benzyloxy}phényl)méthyl]amine et ses sels d'addition d'acides.

**9.** [3'-Pyridylcarbonylimino-(4-{3-[4-(1-méthyl-1-phényléthyl)phénoxyméthyl]benzyloxy}phényl)méthyl]amine et ses sels d'addition d'acides.

**10.** Compositions pharmaceutiques avec des substances auxiliaires et/ou des supports classiques, caractérisées en ce qu'elles contiennent un composé selon les revendications 1 à 9.

**11.** Utilisation de composés de formule générale

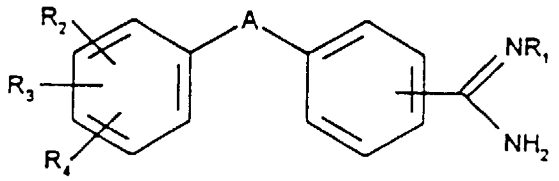

(I)

dans laquelle

A représente le groupe

$$-X_1-C_mH_{2m}-X_2- \qquad (II)$$

(m est égal à un nombre de 2 à 6)

ou

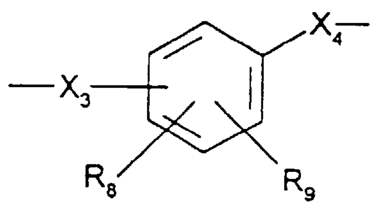

(III);

$X_1$ représente O, NH ou $NCH_3$,

$X_2$ représente O, NH, $NCH_3$ ou

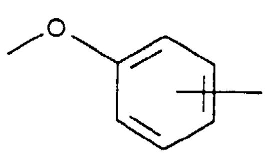

$X_3$ est $-X-C_nH_{2n}-$;

$X_4$ est $-C_nH_{2n}-X-$(n = 1 ou 2, X = O, NH ou $NCH_3$);

$R_1$ est OH, CN, $COR_{12}$, $COOR_{12}$ et CHO;

$R_2$ représente Br, Cl, F, $CF_3$, OH, alkyle en $C_1$-$C_6$, cycloalkyle en $C_5$-$C_7$, aryle, O-aryle, $CH_2$-aryle, $CR_5R_6$-aryle, $C(CH_3)_2$-$R_7$,
et aussi H, lorsque A est un reste de formule III ou lorsque $X_2$ est un reste de formule IV,
et aussi alcoxy en $C_1$-$C_6$, lorsque A est le groupe II, $X_1$ et m ont la signification indiquée ci-dessus et $X_2$ représente NH, $NCH_3$ ou le groupe IV, ou lorsque A représente le groupe III, $X_3$ a la signification indiquée ci-dessus et, dans $X_4$, X est NH ou $NCH_3$;

$R_3$ représente H, alkyle en $C_1$-$C_6$, OH, Cl, F, et aussi alcoxy en $C_1$-$C_6$ lorsque $R_2$ est aryle, O-aryle, $CH_2$-aryle, $CR_5R_6$-aryle ou $C(CH_3)_2$-$R_7$ ou lorsque $X_2$ représente le groupe IV;

| | |
|---|---|
| $R_2$ et $R_3$ | peuvent aussi représenter ensemble un noyau aromatique ou hétéroaromatique condensé; |
| $R_4$ | est H ou alkyle en $C_1$-$C_6$; |
| $R_5$ | est alkyle en $C_1$-$C_4$, $CF_3$, $CH_2OH$, COOH ou COO(alkyle en $C_1$-$C_4$); |
| $R_6$ | est H, alkyle en $C_1$-$C_4$ ou $CF_3$; |
| $R_5$ et $R_6$ | peuvent aussi former ensemble un groupe alkylène en $C_4$-$C_6$; |
| $R_7$ | est $CH_2OH$, COOH, COO(alkyle en $C_1$-$C_4$), $CONR_{10}R_{11}$ ou $CH_2NR_{10}R_{11}$; |
| $R_8$, $R_9$ | sont H, Br, Cl, F, OH, alkyle en $C_1$-$C_6$ ou alcoxy en $C_1$-$C_6$; |
| $R_{10}$ | est H, alkyle en $C_1$-$C_6$, phényle, phényl(alkyle en $C_1$-$C_6$), $COR_{12}$, $COOR_{12}$, CHO, $CONH_2$, $CONHR_{12}$, $SO_2$-(alkyle en $C_1$-$C_6$), $SO_2$-phényle, le reste phényle pouvant être substitué une ou plusieurs fois par Cl, F, $CF_3$, alkyle en $C_1$-$C_4$, OH, alcoxy en $C_1$-$C_4$; |
| $R_{11}$ | est ou alkyle en $C_1$-$C_6$; |
| $R_{10}$ et $R_{11}$ | peuvent aussi représenter ensemble un groupe alkylène en $C_4$-$C_6$; |
| $R_{12}$ | est alkyle en $C_1$-$C_6$, cycloalkyle en $C_5$-$C_7$, aryle, hétéroaryle, aralkyle ou hétéroaryl(alkyle en $C_1$-$C_6$), les groupes aryle ou hétéroaryle pouvant être substitués une ou plusieurs fois par Cl, F, $CF_3$, alkyle en $C_1$-$C_4$, OH ou alcoxy en $C_1$-$C_4$; |

pour la préparation de médicaments destinés au traitement de maladies dans lesquelles on peut utiliser thérapeutiquement des antagonistes de $LBT_4$.

**12.** Utilisation de composés selon la revendication 11 pour la préparation d'un médicament destiné au traitement de maladies dans lesquelles interviennent des processus inflammatoires et/ou allergiques, en particulier l'arthrite, l'asthme, les broncho-pneumopathies chroniques obstructives, le psoriasis, la colite ulcéreuse, la maladie d'Alzheimer, le choc, l'athérosclérose, la sclérose en plaques, et au traitement des gastropathies induites par des anti-inflammatoires non stéroïdiens, ainsi que des métastases et de la leucémie myéloïde chronique.

**13.** Procédé de préparation de composés selon les revendications 1 à 9, caractérisé en ce que

a) on fait réagir une amidine de formule

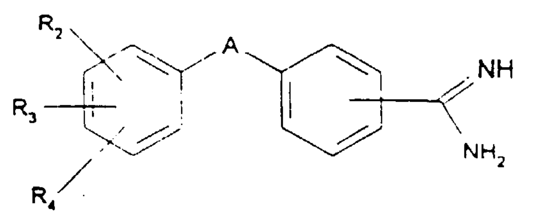

(V)

avec un composé de formule

$$L - R_1' \qquad (VI)$$

où, dans (V), A, $R_2$, $R_3$ et $R_4$ ont la signification donnée ci-dessus, $R_1$' a la même signification que $R_1$ à l'exception de OH, et L est un groupe partant nucléophile comme un atome d'halogène (comme Cl, Br) ou un groupe acyloxy, ou en ce que
b) on fait réagir un composé de formule

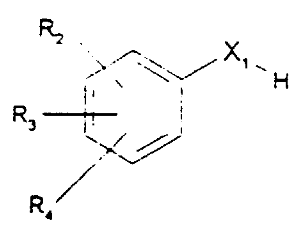

(VII)

(dans laquelle $R_2$, $R_3$, $R_4$ et $X_1$ ont la signification indiquée ci-dessus) avec un dérivé de benzamidine de formule

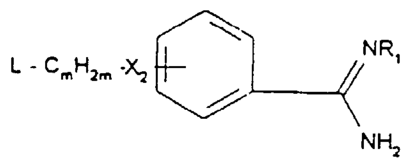

(VIII)

ou

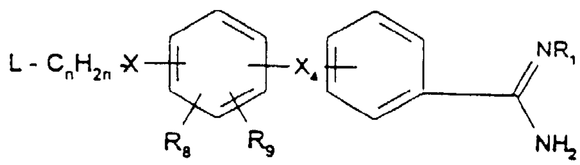

(IX)

(dans lesquelles L, m, n, X, $X_2$, $X_4$, $R_1$, $R_8$ et $R_9$ ont la signification indiquée ci-dessus), ou en ce que
c) on fait réagir un composé de formule

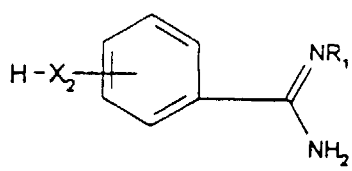

(X)

($R_1$ et $X_2$ ont la signification indiquée ci-dessus) avec un composé de formule

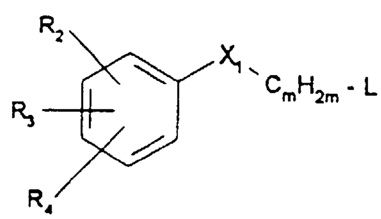

(XI)

ou

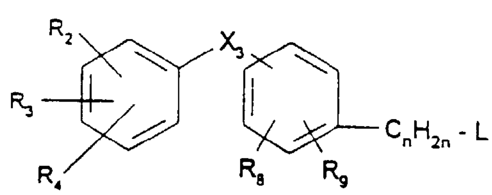

(XII)

(dans lesquelles L, m, n, $R_2$, $R_3$, $R_4$, $R_8$, $R_9$, $X_1$ et $X_3$ ont la signification donnée ci-dessus)
ou en ce que
d) pour préparer des composés de formule I dans lesquels $R_1$ est OH, on fait réagir un nitrile de formule

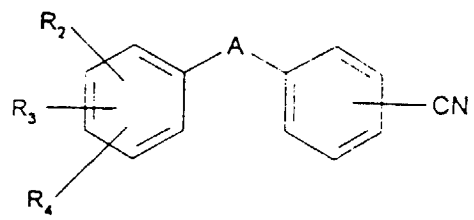

(XIII)

dans laquelle A, $R_2$, $R_3$ et $R_4$ ont la signification indiquée ci-dessus,
avec de l'hydroxylamine,

et, à partir des produits obtenus, selon leur type, on prépare éventuellement des antipodes optiques ou des paires d'antipodes diastéréoisomères,
ou en ce que l'on prépare des sels d'addition d'acides à partir des bases obtenues ou des bases libres à partir des sels d'addition d'acides d'abord obtenus.